# EUROPEAN PATENT SPECIFICATION

(11) **EP 4 065 178 B1**
(45) Date of publication and mention of the grant of the patent: **14.02.2024**
(21) Application number: 20811634.3
(22) Date of filing: 27.11.2020
(51) Int. Cl.: A61L 2/10, B01J 19/12

(54) **ULTRAVIOLET RADIATION-BASED SURFACE DISINFECTION AND/OR SURFACE PROCESSING SYSTEM**
AUF ULTRAVIOLETTSTRAHLUNG BASIERENDES OBERFLÄCHENDESINFEKTIONS- UND/ODER OBERFLÄCHENBEHANDLUNGSSYSTEM
SYSTÈME DE DÉSINFECTION DE SURFACE ET/OU DE TRAITEMENT DE SURFACE À BASE DE RAYONNEMENT ULTRAVIOLET

(30) Priority: 27.11.2019 NL 2024320
(43) Date of publication of application: 05.10.2022
(73) Proprietor: Dinnissen B.V., 5975 NB Sevenum (NL)
(72) Inventor: CLEVEN, Christiaan Gerard Pieter, 5975 NB Sevenum (NL); KUIJPERS, Wouter Theo Alex Robert, 5975 NB Sevenum (NL); EGELMEERS, Luc Berry Anne, 5975 NB Sevenum (NL)
(74) Representative: IPecunia
(86) International application number: PCT/EP2020/083610
(87) International publication number: WO 2021/105342

(56) References cited:
- EP-A1- 0 069 767
- EP-B1- 0 069 767
- WO-A2-2007/061401
- GB-A- 2 424 877
- US-A1- 2003 049 809
- US-A1- 2013 008 857
- US-B1- 6 309 611

## Description

### FIELD OF THE INVENTION

The present invention relates to an ultraviolet radiation-based surface disinfection and/or surface processing system that is adapted to disinfect outer surfaces of solid particles of a stock of solid particles with ultraviolet radiation and optionally initiate chemical processes at outer surfaces of solid particles of a stock of solid particles with ultraviolet radiation

### BACKGROUND OF THE INVENTION

It is well known that radiation having wavelengths in the ultraviolet (UV) spectrum, which is defined as wavelengths between 10 nm and 405 nm, does not deeply penetrate a target that is irradiated with such radiation. Therefore, UV radiation is commonly used for treating the outer surface of the target being irradiated. Examples of such surface treatments include disinfection or sterilization of the outer surface and/or initiation of chemical processes at the outer surface, so-called UV curing. For UV curing typically UV radiation having a wavelength between 365 nm and 405 nm is used. Examples of such systems are shown in US 2013/008857, EP 0 069 767, WO 2007/061401, GB 2 424 877, US2003049809, US6309611.

For disinfection or sterilization of the outer surface a so-called germicidal range of the UV spectrum can be used. The germicidal range has wavelengths between 100 nm and 300 nm. UV radiation having wavelengths in the latter range is generally referred to as UV-C radiation. UV-C radiation having wavelengths between 200 nm and 300 nm is considered germicidal against microorganisms such as for example bacteria, viruses, protozoa, moulds, yeasts and algae. It is known in the art that the germicidal effect is due to changes in at least one of the deoxyribonucleic acid (DNA) and ribonucleic acid (RNA) structures in cells of microorganisms of at least one of the kinds mentioned above due to irradiation using UV-C radiation. These changes in at least one of the DNA and RNA structures render the cells and therefore the microorganisms incapable of replicating. A cell that cannot replicate is construed to be dead since such a cell is unable to multiply to infectious numbers within a host. It is known in the art that the highest germicidal effect can be obtained using UV-C radiation having wavelengths between 250 nm and 270 nm, with the peak wavelength for germicidal activity being 265 nm. At wavelengths above 300 nm, the germicidal effect of the UV-C radiation is negligible.

While UV radiation can effectively be used for processes as the ones mentioned above, there are limitations for its use. A limitation is that UV radiation can only effectively be applied in a "line-of-sight" fashion, i.e. only areas of a surface that are within direct sightlines of the UV radiation source can effectively be irradiated with UV radiation. Areas of a surface that cannot directly be irradiated with UV radiation are commonly referred to as shadow areas. In these shadow areas the abovementioned surface treatments including disinfection or sterilization of the outer surface and/or initiation of chemical processes at the outer surface cannot be performed effectively or even not at all.

In view of the aforementioned limitation regarding the application of UV-C radiation, the person skilled in the art will appreciate that known ultraviolet radiation-based surface disinfection and/or sterilization systems cannot advantageously be used to adequately disinfect or sterilize outer surfaces of solid particles that are arranged for example as stocks of solid particles. Due to the arrangement of the solid particles in such a stock, it will be clear that only areas of the outer surfaces of the solid particles that form an outer layer of the stock can directly be irradiated with UV-C radiation. Therefore, many shadow areas will exist, and many solid particles will at least partially be shielded from UV-C radiation by at least one of surrounding solid particles of the stock and parts of the system itself.

Based on the above, there is a need for an ultraviolet radiation-based surface disinfection and/or surface processing system that is adapted to enable enhanced disinfection of outer surfaces of solid particles that are arranged as stocks of solid particles using UV-C radiation and optionally to enable enhanced initiation of chemical processes at outer surfaces of solid particles of stocks of solid particles with UV-C radiation.

### SUMMARY OF THE INVENTION

It is an object of the present invention to provide an ultraviolet radiation-based surface disinfection and/or surface processing system that is adapted to disinfect outer surfaces of solid particles of a stock of solid particles with ultraviolet radiation and optionally initiate chemical processes at outer surfaces of solid particles of a stock of solid particles with ultraviolet radiation that pre-empts or at least reduces at least one of the abovementioned and/or other disadvantages associated with ultraviolet radiation-based surface disinfection and/or sterilization systems known in the art.

Aspects of the present invention are set out in the accompanying independent and dependent claims. Features from the dependent claims may be combined with features from the independent claim as appropriate and not merely as explicitly set out in the claims.

At least one of the abovementioned objects is achieved by an ultraviolet radiation-based surface disinfection and/or surface processing system that is adapted to disinfect outer surfaces of solid particles of a stock of solid particles with ultraviolet radiation and/or to initiate chemical processes at outer surfaces of solid particles of a stock of solid particles with ultraviolet radiation, said system comprising a process chamber that is configured to contain a stock of solid particles, wherein each one of the solid particles of the stock of solid particles has a volume that is equal to or less than 10 cm³, the process chamber being provided with:
- a first activation arrangement and a second activation arrangement that are arranged with respect to each other to, when said system is in use, establish fluidization of solid particles of the stock of solid particles in a fluidization zone between the first activation arrangement and the second activation arrangement, wherein fluidized solid particles are spaced apart in the fluidization zone; and
- an ultraviolet radiation unit that is configured to emit ultraviolet radiation having a wavelength in a range of 200 nm - 300 nm, wherein the ultraviolet radiation unit is arranged to at least partially irradiate the outer surfaces of at least the fluidized solid particles in the fluidization zone.

As a result of the fact that the fluidized solid particles of the stock of solid particles are spaced apart and tumbled around in the fluidization zone in the process chamber, the abovementioned shadow areas can be reduced. Consequently, the outer surfaces of the fluidized solid particles can be irradiated more effectively by the UV radiation having a wavelength in the range of 200 nm - 405 nm that is emitted by the ultraviolet radiation unit. The person skilled in the art will appreciate that at least one of an appropriate shape of the process chamber, i.e. a shape that for example allows improved positioning of the ultraviolet radiation unit with respect to the fluidization zone, and an appropriate condition of the inner surface of the process chamber, i.e. a suitable surface roughness and/or a suitable kind of material used to form the inner surface, can also improve irradiation efficiency of the outer surfaces of at least the fluidized solid particles in the fluidization zone. In at least one of the ways described above, the system according to the invention can enable enhanced disinfection of outer surfaces of at least the fluidized solid particles and optionally enhanced initiation of chemical processes at the outer surfaces of at least the fluidized solid particles.

The person skilled in the art will appreciate that any suitable number of ultraviolet radiation units can be applied in order to meet the specific requirements of the system according to the invention.

In addition, the person skilled in the art will appreciate that as all solid particles of the stock of solid particles contained in the process chamber can at least be fluidized once, i.e. they can at least once be brought into the fluidization zone, and irradiated by for example UV-C radiation having a wavelength between 200nm and 300 nm, the system according to invention can achieve a predetermined log reduction of the microorganisms present at the outer surfaces of the solid particles of the stock of solid particles contained in the process chamber. The person skilled in the art will appreciate that the log reduction is a mathematical term that is used to express the relative number of living microorganisms that are eliminated by disinfection using for example UV-C radiation. The log reduction can be expressed as follows:
log reduction = log10 (*N₀*/*N*), wherein *N₀* is the colony forming units of the microorganisms before exposure to UV-C radiation, and *N* is the colony forming units of the microorganisms after exposure to UV-C radiation. For example, a 1-log reduction corresponds to inactivating 90% of a target microorganism with the microorganism count being reduced by a factor of 10. From the latter it will be clear that a 2-log reduction will see a 99% reduction, or microorganism reduction by a factor of 100, a 3-log reduction will see a 99.9% reduction, or microorganism reduction by a factor of 1,000, a 4-log reduction will see a 99.99% reduction, or microorganism reduction by a factor of 10,000, a 5-log reduction will see a 99.999% reduction, or microorganism reduction by a factor of 100,000, and a 6-log reduction will see a 99.9999% reduction, or microorganism reduction by a factor of 1,000,000. For example, for disinfection of food products, typically a 4-log reduction is preferred.

The person skilled in the art will appreciate that the first activation arrangement and the second activation arrangement can only establish a proper fluidization zone with fluidized solid particles if each one of the solid particles has a volume that is equal to or less than 10 cm³. In addition, the person skilled in the art will appreciate that the solid particles should be dry enough to allow fluidization thereof. It is noted that it depends on the type of solid particles that need to be processed using the system according to the invention how the phrase "dry enough" should be construed as it will have different implications for solid particles that are for example powder particles, granules or reduced food parts. The latter can for example be reduced herb parts of one kind or of different kinds.

The system according to the invention can also be used to process a stock of solid particles comprising a combination of the aforementioned types of solid particles, for example reduced food parts of any kind and powder particles and/or granules of any kind.

In addition, the person skilled in the art will appreciate that the first activation arrangement and the second activation arrangement can be constructed in any suitable way as long as they enable fluidization of the solid particles of the stock of solid particles contained in the process chamber of the system according to the invention.

Furthermore, the person skilled in the art will appreciate that the system according to the present invention allows processing of the solid particles using a continuous process or a batch process.

The first activation arrangement comprises a first elongated axle that is provided with a first set of paddles and the second activation arrangement comprises a second elongated axle that is provided with a second set of paddles, wherein, when the system is in use, the first set of paddles and the second set of paddles are configured to interact with the solid particles of the stock of solid particles in the process chamber, and the first elongated axle and the second elongated axle are configured and arranged to simultaneously rotate in opposite directions.

The person skilled in the art will appreciate that the abovementioned implementations of the first activation arrangement and of the second activation arrangement are an example of advantageous implementations thereof. As mentioned above, the first activation arrangement and the second activation arrangement can be constructed in any suitable way, for example using ribbons or the above-mentioned paddles, as long as they enable fluidization of the solid particles of the stock of solid particles contained in the process chamber of the system according to the invention.

The person skilled in the art will appreciate that the first elongated axle and the second elongated axle can be arranged in any suitable way with respect to each other as long as a fluidization zone can be established when the system is in use. The first elongated axle and the second elongated axle are arranged parallel with respect to each other.

Furthermore, the person skilled in the art will appreciate that the first set of paddles of the first elongated axle and the second set of paddles of the second elongated axle can be configured to preserve the integrity of the outer surfaces of the solid particles processed using the system according to the invention. As an example, buyers of reduced food parts, for example finely chopped vegetables, value a well-preserved integrity of the outer surfaces thereof. Therefore, an advantage of the system according to the present invention is that it allows processing of solid particles of which a well-preserved integrity of their outer surfaces is important to buyers of these goods.

In addition, the person skilled in the art will appreciate that the paddles of the first set of paddles and the paddles of the second set of paddles can be arranged in any suitable way along the first elongated axle and along the second elongated axle respectively as long as the paddles of both the first set of paddles and of the second set of paddles allow fluidization of the solid particles of the stock of solid particles contained in the process chamber and preferably allow the preservation of the integrity of the outer surfaces of these solid particles.

Moreover, the person skilled in the art will appreciate that besides the establishment of the above-mentioned fluidization zone and preferably the preservation of the integrity of the outer surfaces of the solid particles, the combination of the first elongated axle that is provided with the first set of paddles and the second elongated axle that is provided with the second set of paddles also allows fast mixing of different kinds of solid particles of the same or different types that are dry enough. In this way, mixtures can be obtained comprising for example powder particles and granules, powder particles of different kinds, granules of different kinds or reduced food parts of different kinds. Moreover, the mixtures of solid particles thus obtained can have a high homogeneity. Based on the foregoing, the person skilled in the art will appreciate that the system according to the invention can be construed as a mixer that allows the outer surfaces of solid particles that are arranged as stocks of solid particles to be disinfected using UV-C radiation and optionally to enable enhanced initiation of chemical processes at the outer surfaces of the solid particles of stocks of solid particles with UV-C radiation while being mixed in the fluidization zone.

In addition, the person skilled in the art will appreciate that the system of the present invention is configured to achieve a fluidization zone that preferably has a Froude number of at least 0.8. If the Froude number is less than 0.8, there cannot be sufficient fluidization of the solid particles because not enough space between the solid particles can be established. In that case, only limited mixing or practically no mixing at all can be achieved. Furthermore, the person skilled in the art will appreciate that the Froude number can be determined considering the speed at which the first elongated axle of the first activation arrangement and the second elongated axle of the second activation arrangement are rotated, the so-called tip velocities of the paddles of the respective first set of paddles and second set of paddles, and the dimensions of the process chamber.

In an embodiment of the system according to the invention, the ultraviolet radiation unit comprises an ultraviolet radiation source that has a longitudinal center line, wherein the longitudinal center line of the ultraviolet radiation source is arranged parallel to a longitudinal center line of the first elongated axle and a longitudinal center line of the second elongated axle. The aforementioned arrangement of the ultraviolet radiation source with respect to the first elongated axle and the second elongated axle is an example of an advantageous arrangement that allows the fluidized solid particles in the fluidization zone to be effectively irradiated with UV radiation. The person skilled in the art will appreciate that the ultraviolet radiation source can be arranged with respect to the first elongated axle and the second elongated axle in any suitable way as long as the fluidized solid particles in the fluidization zone can be effectively irradiated with UV radiation. Furthermore, the person skilled in the art will appreciate that any suitable number of ultraviolet radiation sources can be applied in order to meet the specific requirements of the system according to the invention.

In an embodiment of the system according to the invention, the ultraviolet radiation unit comprises an ultraviolet radiation source that has a longitudinal center line, wherein the longitudinal center line of the ultraviolet radiation source is arranged in a first direction that is at a non-zero angle with both a longitudinal center line of the first elongated axle and a longitudinal center line of the second elongated axle. The person skilled in the art will appreciate that this arrangement of the ultraviolet radiation source with respect to the first elongated axle and the second elongated axle is an example of another advantageous arrangement that allows the fluidized solid particles in the fluidization zone to be effectively irradiated with UV radiation.

As mentioned above, the person skilled in the art will appreciate that any suitable number of ultraviolet radiation sources can be applied in order to meet the specific requirements of the system according to the invention.

In an embodiment of the system according to the invention, the ultraviolet radiation unit comprises an ultraviolet radiation source that has a circular or a square cross-section. The person skilled in the art will appreciate that the ultraviolet radiation source can have any suitable cross-section that allows the fluidized solid particles in the fluidization zone to be effectively irradiated with UV radiation. The person skilled in the art will appreciate that the circular and square cross-sections are advantageous for arranging ultraviolet radiation sources in a matrix configuration, for example an *N*×*M* matrix, with N and M being a counting number. A matrix configuration of the ultraviolet radiation sources is an example of an advantageous arrangement that allows the fluidized solid particles in the fluidization zone to be effectively irradiated with UV radiation.

In an embodiment of the system according to the invention, the ultraviolet radiation source is one of a mercury-arc lamp, a light emitting diode (LED) and a laser diode (LD) being adapted to emit ultraviolet radiation having a wavelength in the range of 200 nm - 405 nm. The person skilled in the art will appreciate that the aforementioned types of radiation sources are examples of advantageous types of radiation sources to provide UV radiation having wavelengths between 200 nm and 405 nm. As mentioned above, the highest germicidal effect can be obtained using UV-C radiation having wavelengths between 250 nm and 270 nm, with the peak wavelength for germicidal activity being 265 nm.

Moreover, the person skilled in the art will appreciate that depending on the specific requirements of the system according to the invention, the ultraviolet radiation unit can be provided with any suitable combination of the aforementioned types of radiation sources.

In an embodiment of the system according to the invention, the ultraviolet radiation source is arranged inside a housing that is transparent at least for ultraviolet radiation having a wavelength in the range of 200 nm - 405 nm. The person skilled in the art will appreciate that each ultraviolet radiation source can be arranged inside a dedicated housing but that it is also possible that multiple ultraviolet radiation sources are arranged within one common housing. The housing of course needs to be adapted to allow the UV radiation with a wavelength between 200 nm and 405 nm to be transmitted through the housing, i.e. not to be absorbed by or not to be blocked by the housing, in order to irradiate the fluidized solid particles that are spaced apart in the fluidization zone in the process chamber of the system according to the invention. The housing can comprise for example acrylates that are adapted to allow the UV radiation to be transmitted through the housing. It is also possible that the housing is provided with a window that is adapted to allow the UV radiation to be transmitted through the housing.

In an embodiment of the system according to the invention, the process chamber is provided with an air knife that is configured and arranged to remove solid particles from the housing. The person skilled in the art will appreciate that in the event that the solid particles are for example granules, there generally will be little dust in the process chamber upon processing the granules. The term "dust" is to be construed as residue of the granules or even the granules themselves in the case of very small granules. In that case, the application of an air knife can be sufficient to remove the dust from the housing. The person skilled in the art will appreciate that depending on the actual arrangement and number of the housings used, one air knife or multiple air knives can be used to clean the housing or housings in order to enable efficient irradiation of the fluidized solid particles that are spaced apart in the fluidization zone in the process chamber.

In an embodiment of the system according to the invention, the housing is a rotatable housing. The person skilled in the art will appreciate that in the event that the solid particles are for example powder particles, there will generally be a lot of dust in the process chamber upon processing the powder particles. In that case, the application of at least one of an air knife and a rotatable housing can achieve efficient removal of the powder particles from the housing. The housing can be arranged to be rotatable around the ultraviolet radiation source or sources stationary arranged inside the housing.

In an embodiment of the system according to the invention, the process chamber is provided with a radiation intensity detection unit that is configured and arranged to perform a measurement of an intensity of the ultraviolet radiation in the fluidization zone during a predefined time interval and to determine based on said measurement if a dose of the ultraviolet radiation in the fluidization zone is above a predetermined threshold to achieve disinfection of the outer surfaces of the fluidized solid particles in the fluidization zone and/or initiation of chemical processes at the outer surfaces of the fluidized solid particles in the fluidization zone, wherein the predetermined threshold for the dose of the ultraviolet radiation is dependent on at least one of a type of microorganisms that are present on the outer surfaces of the solid particles, a distance between the ultraviolet radiation unit and the fluidized solid particles in the fluidization zone, a volume of the process chamber, the volume of the solid particles, and the outer surface area of the solid particles.

The person skilled in the art will appreciate that the radiation intensity detection unit allows checking of the condition of the ultraviolet radiation unit. In this way, it can be monitored when maintenance, e.g. replacement of the ultraviolet radiation source or cleaning of the ultraviolet radiation unit, is due. Especially in the case that the system according to the invention is configured for continuous processing, the radiation intensity detection unit can be used to check if the ultraviolet radiation unit remains within the required specifications during operation of the system according to the invention. In the case that the system according to the invention is configured for batch processing, the radiation intensity detection unit can be used to check before the start of each batch if the ultraviolet radiation unit is within the required specifications. If this is the case, the batch can be started. If this is not the case, appropriate maintenance needs to be done before starting the batch.

The person skilled in the art will appreciate that any suitable kind of radiation intensity detection unit can be used. Moreover, the person skilled in the art will appreciate that any suitable number of radiation intensity detection units can be applied in order to meet the specific requirements of the system according to the invention.

In an embodiment of the system according to the invention, the process chamber has an inner surface having an arithmetic average surface roughness Ra that is equal to or less than 0.8 µm. The person skilled in the art will appreciate that in this way reflection of the UV radiation from uncovered parts of the inner surface of the process chamber can be enhanced. The reflected UV radiation can contribute to the effective irradiation of the fluidized solid particles that are spaced apart in the fluidization zone in the process chamber of the system according to the invention.

It is noted that in the case that the inner surface of the process chamber is made of stainless steel, 20-25% UV-C radiation can be reflected depending on the exact composition of the stainless steel and the value of Ra. By applying mirrored aluminum foil at the inner surface of the process chamber, 80-90% UV-C radiation can be reflected depending on the exact composition of the aluminum foil and the value of Ra. By applying an expanded Polytetrafluoroethylene (ePTFE) coating or Polytetrafluoroethylene (PTFE) sheeting material, 80-98% UV-C radiation can be reflected depending on the exact composition of the ePTFE coating or the PTFE sheeting material and the respective values of Ra. Based on the foregoing, the person skilled in the art will appreciate that it is advantageous to apply at least one of mirrored aluminum foil, ePTFE coating and PTFE sheeting material instead of stainless steel at the inner surface of the process chamber to enable sufficient reflection of UV-C radiation.

Furthermore, it is noted that the person skilled in the art will appreciate that Ra can be measured in accordance with standard ISO 4287:1997 Geometrical Product Specifications (GPS) - Surface texture: Profile method - Terms, definitions and surface texture parameters.

In an embodiment of the system according to the invention, the process chamber is provided with a surface treatment application unit that is configured and arranged to apply a surface treatment composition to the outer surfaces of the fluidized solid particles that are in the fluidization zone.

It will be clear to the person skilled in the art that a suitable surface treatment composition can be applied to the outer surfaces of the solid particles that are in the fluidization zone before or after irradiation with UV radiation. The person skilled in the art will appreciate that if a suitable surface treatment composition is applied to the outer surfaces of the solid particles after they have been disinfected or sterilized by UV-C irradiation, it is possible to prevent the outer surfaces from subsequently becoming contaminated. For example, reduced food parts that have been processed using the system according to the invention but whose outer surfaces have not been treated using a suitable surface treatment composition, can be susceptible to a subsequent contamination with salmonella. By applying a suitable surface treatment composition on the outer surfaces of the reduced food parts after irradiation thereof with UV-C radiation, the risk of such subsequent contamination of the outer surfaces can at least be reduced.

In addition, the person skilled in the art will appreciate that it is also possible that after application of a suitable surface treatment composition to the outer surfaces of the solid particles that are in the fluidization zone, said surface treatment composition can be cured using UV radiation having a wavelength between 365 nm and 405 nm.

Furthermore, the person skilled in the art will appreciate that by treating the outer surfaces with a suitable surface treatment composition it is also possible to for example reinstate microorganisms at the outer surfaces of the processed solid particles which not necessarily would have had to be removed and which may even have positive effects.

In an embodiment of the system according to the invention, the surface treatment application unit is a spray unit that is adapted to spray the surface treatment composition on the outer surfaces of the fluidized solid particles that are in the fluidization zone.

The person skilled in the art will appreciate that by using a spray unit the surface treatment composition can advantageously be applied to the outer surfaces of the fluidized solid particles that are in the fluidization zone after disinfection of the outer surfaces of the fluidized solid particles and/or after initiating chemical processes at the outer surfaces of the fluidized solid particles. However, the person skilled in the art will appreciate that any suitable application unit can be used as long as the surface treatment composition can effectively be applied to the outer surfaces of the fluidized solid particles.

The surface treatment composition can comprise a surface sealing composition. The person skilled in the art will appreciate that by sealing the outer surfaces of the fluidized solid particles that are in the fluidization zone after disinfection of the outer surfaces of the fluidized solid particles and/or after initiating chemical processes at the outer surfaces of the fluidized solid particles, the risk of subsequent contamination thereof can even further be reduced.

In an embodiment of the system according to the invention, the process chamber is provided with a folding bottom that is adapted to enable emptying of the process chamber. The person skilled in the art will appreciate that in this way a residual-poor emptying of the process chamber can be achieved.

The process chamber is configured to receive different kinds of solid particles that all have outer surfaces that are to be disinfected using ultraviolet radiation having a wavelength in a range of 200 nm - 300 nm while the solid particles are being mixed in the fluidization zone and/or to be irradiated using ultraviolet radiation having a wavelength in a range of 365 nm - 405 nm to initiate chemical processes thereon while the solid particles are being mixed in the fluidization zone. As mentioned above, the person skilled in the art will appreciate that the system according to the present invention besides irradiating the outer surfaces of the fluidized solid particles of the different kinds, can also be used to enable fast mixing thereof. It is noted that the solid particles can be of the same or of a different type. In this way, mixtures can be obtained comprising for example powder particles and granules, powder particles of different kinds, granules of different kinds or reduced food parts of different kinds. Moreover, the mixtures of solid particles thus obtained can have a high homogeneity.

### BRIEF DESCRIPTION OF THE DRAWINGS

Further features and advantages of the invention will become apparent from the description of the invention by way of exemplary and non-limiting embodiments of an ultraviolet radiation-based surface disinfection and/or surface processing system according to the invention.

The person skilled in the art will appreciate that the described embodiments of the system according to the invention are exemplary in nature only and not to be construed as limiting the scope of protection in any way.

Reference will be made to the figures on the accompanying drawing sheets. The figures are schematic in nature and therefore not necessarily drawn to scale. Furthermore, equal reference numerals denote equal or similar parts. On the attached drawing sheets,
figure 1 shows a schematic cross-sectional view of an exemplary, non-limiting first embodiment of an ultraviolet radiation-based surface disinfection and/or surface processing system according to the invention;
figure 2 shows a schematic top view of the exemplary, non-limiting first embodiment of the ultraviolet radiation-based surface disinfection and/or surface processing system shown in figure 1;
figure 3 shows a schematic top view of an exemplary, non-limiting second embodiment of the ultraviolet radiation-based surface disinfection and/or surface processing system according to the invention;
figure 4 shows a schematic cross-sectional view of an exemplary, non-limiting third embodiment of an ultraviolet radiation-based surface disinfection and/or surface processing system according to the invention;
figure 5 shows a schematic cross-sectional view of an exemplary, non-limiting fourth embodiment of an ultraviolet radiation-based surface disinfection and/or surface processing system according to the invention;
figure 6A shows a schematic front view of an exemplary, non-limiting second embodiment of the first activation arrangement and the second activation arrangement;
figure 6B shows a schematic front view of an exemplary, non-limiting third embodiment of the first activation arrangement and the second activation arrangement; and
figure 6C shows a schematic front view of an exemplary, non-limiting fourth embodiment of the first activation arrangement and the second activation arrangement.

### DETAILED DESCRIPTION OF EMBODIMENTS

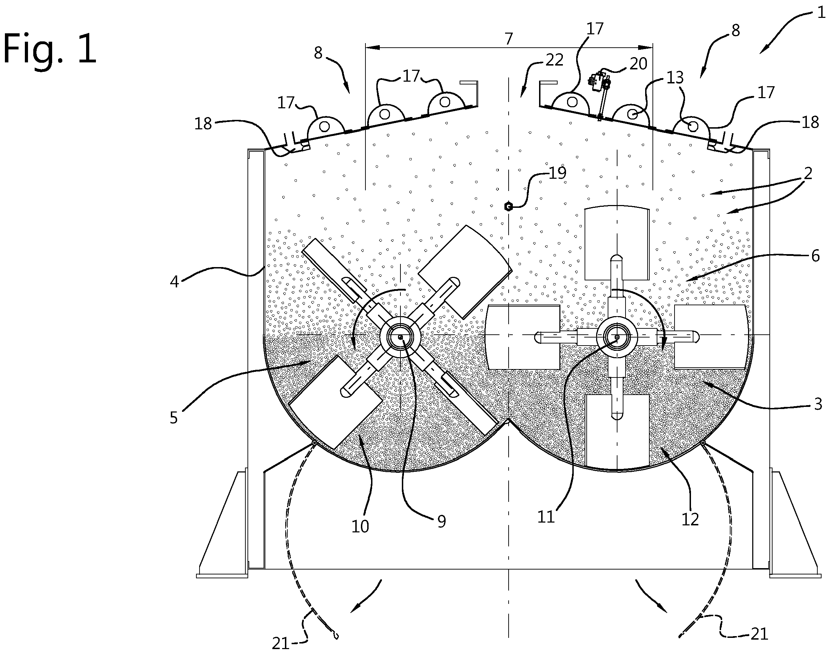

Figure 1 shows a schematic cross-sectional view of an exemplary, non-limiting first embodiment of an ultraviolet radiation-based surface disinfection and/or surface processing system 1 according to the invention. The system 1 is adapted to disinfect outer surfaces of solid particles 2 of a stock 3 of solid particles 2 with ultraviolet (UV) radiation and optionally initiate chemical processes at outer surfaces of solid particles 2 of a stock 3 of solid particles 2 with ultraviolet radiation.

The system 1 comprises a process chamber 4 that is configured to contain a stock 3 of solid particles 2. The process chamber 4 is provided with a first activation arrangement 5 and a second activation arrangement 6 that are arranged with respect to each other to, when the system 1 is in use, establish fluidization of solid particles 2 of the stock 3 of solid particles 2 in a fluidization zone 7 that is established between the first activation arrangement 5 and the second activation arrangement 6. The fluidized solid particles 2 are spaced apart in the fluidization zone 7.

The person skilled in the art will appreciate that the first activation arrangement 5 and the second activation arrangement 6 of the system 1 can only establish a proper fluidization zone 7 with fluidized solid particles 2 if each one of the solid particles 2 has a volume that is equal to or less than 10 cm³. In addition, the person skilled in the art will appreciate that the solid particles 2 should be dry enough to allow fluidization thereof. It is noted that it depends on the type of solid particles 2 that need to be processed using the system 1 how the phrase "dry enough" should be construed as it will have different implications for solid particles that are for example powder particles, granules or reduced food parts. The latter can for example be reduced herb parts of one kind or of different kinds. The system 1 can also be used to process a stock 3 of solid particles 2 comprising a combination of the aforementioned types of solid particles 2, for example reduced food parts of any kind and powder particles and/or granules of any kind.

It will be clear that the first activation arrangement 5 and the second activation arrangement 6 of the system 1 can be constructed in any suitable way as long as they enable fluidization of the solid particles 2 of the stock 3 of solid particles 2 contained in the process chamber 4. Figures 1, 4 and 5 show an exemplary, non-limiting first embodiment of the first activation arrangement 5 and the second activation arrangement 6. In accordance with said first embodiment, the first activation arrangement 5 comprises a first elongated axle 9 that is provided with a first set of paddles 10 having a first exemplary shape and the second activation arrangement 6 comprises a second elongated axle 11 that is provided with a second set of paddles 12 having said first exemplary shape. When the system 1 is in use, the first set of paddles 10 and the second set of paddles 12 are configured to interact with the solid particles 2 of the stock 3 of solid particles 2 in the process chamber 4, and the first elongated axle 9 and the second elongated axle 11 are configured and arranged to simultaneously rotate in opposite directions. The opposite rotation directions of the first elongated axle 9 and the second elongated axle 11 are indicated in figure 1 with the thick curved arrows.

As shown in figure 1, the first elongated axle 9 and the second elongated axle 11 are arranged parallel with respect to each other. It will be clear that the first elongated axle 9 and the second elongated axle 11 can be arranged in any suitable way with respect to each other as long as the fluidization zone 7 can be established.

The first set of paddles 10 of the first elongated axle 9 and the second set of paddles 12 of the second elongated axle 11 can be configured to preserve the integrity of the outer surfaces of the solid particles 2 processed using the system 1. In addition, the paddles of the first set of paddles 10 and the paddles of the second set of paddles 12 can be arranged in any suitable way along the first elongated axle 9 and along the second elongated axle 11 respectively as long as the paddles of both the first set of paddles 10 and of the second set of paddles 12 allow fluidization of the solid particles 2 of the stock 3 of solid particles 2 contained in the process chamber 4 and preferably allow the preservation of the integrity of the outer surfaces of these solid particles 2.

Moreover, the person skilled in the art will appreciate that besides the establishment of the above-mentioned fluidization zone 7, the combination of the first elongated axle 9 that is provided with the first set of paddles 10 and the second elongated axle 11 that is provided with the second set of paddles 12 also allows fast mixing of different kinds of solid particles 2 of the same or different types that are dry enough. In this way, mixtures can be obtained comprising for example powder particles and granules, powder particles of different kinds, granules of different kinds or reduced food parts of different kinds. Moreover, the mixtures of solid particles thus obtained can have a high homogeneity.

In addition, the person skilled in the art will appreciate that the system 1 is configured to achieve a fluidization zone 7 that preferably has a Froude number of at least 0.8. If the Froude number is less than 0.8, there cannot be sufficient fluidization of the solid particles 2 because not enough space between the solid particles 2 can be established. In that case, only limited mixing or practically no mixing at all can be achieved. Furthermore, the person skilled in the art will appreciate that the Froude number can be determined considering the speed at which the first elongated axle 9 of the first activation arrangement 5 and the second elongated axle 11 of the second activation arrangement 6 are rotated, the so-called tip velocities of the paddles of the respective first set of paddles 10 and second set of paddles 12, and the dimensions of the process chamber 4.

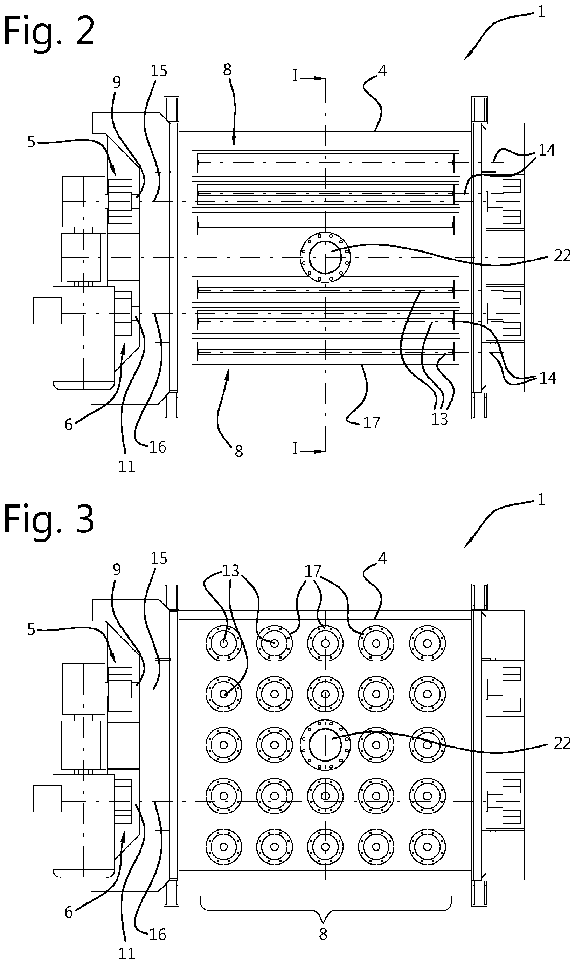

Figure 1 further shows that the process chamber 4 of the system 1 comprises ultraviolet radiation units 8. The latter are configured to emit UV-C radiation having a wavelength in a range of 200 nm - 300 nm and are arranged to at least partially irradiate the outer surfaces of at least the fluidized solid particles 2 in the fluidization zone 7. As can be seen in figure 2, which provides a schematic top view of the system 1 shown in figure 1, the ultraviolet radiation units 8 can be arranged at opposite sides of an inlet opening 22 that is adapted to introduce solid particles 2 that are to be processed with the system 1 into the process chamber 4.

As a result of the fact that the fluidized solid particles 2 of the stock 3 of solid particles 2 are spaced apart and tumbled around in the fluidization zone 7 in the process chamber 4, so-called shadow areas can be reduced. Consequently, the outer surfaces of the fluidized solid particles 2 can be irradiated more effectively by the UV radiation that is emitted by the ultraviolet radiation units 8. As all solid particles 2 of the stock 3 of solid particles 2 contained in the process chamber 4 can at least be fluidized once, i.e. they can at least once be brought into the fluidization zone 7, and irradiated by for example UV-C radiation, the system 1 can achieve a predetermined log reduction of the microorganisms present at the outer surfaces of the solid particles 2 of the stock 3 of solid particles 2 contained in the process chamber 4.

The person skilled in the art will appreciate that enhanced disinfection of outer surfaces of at least the fluidized solid particles 2 and optionally enhanced initiation of chemical processes at the outer surfaces of at least the fluidized solid particles 2 can be achieved by providing the process chamber 4 with an appropriate shape, i.e. a shape that for example allows improved positioning of the ultraviolet radiation units 8 with respect to the fluidization zone 7. The process chamber 4 of the system 1 shown in figure 1 has a top part comprising slanted portions, each of which are provided with one ultraviolet radiation unit 8. This arrangement of the ultraviolet radiation units 8 can improve the irradiation efficiency of the solid particles 2 in the fluidization zone 7. Another way to enhance disinfection of outer surfaces of at least the fluidized solid particles 2 and optionally enhanced initiation of chemical processes at the outer surfaces of at least the fluidized solid particles 2 can be achieved by appropriately conditioning an inner surface of the process chamber 4 that faces the solid particles 2. Appropriately conditioning of the inner surface can for example be achieved by at least one of providing a suitable kind of material to form the inner surface of the process chamber 4 and by providing the material that forms the inner surface of the process chamber 4 with a surface roughness Ra that is equal to or less than 0.8 µm. In this way, reflection of the UV radiation from uncovered parts of the inner surface of the process chamber 4 can be enhanced. The reflected UV radiation can contribute to the effective irradiation of the fluidized solid particles 2 that are spaced apart and tumbled around in the fluidization zone 7 in the process chamber 4.

Figure 2 shows that the ultraviolet radiation units 8 of the system 1 comprise ultraviolet radiation sources 13 each having longitudinal central axes 14. In accordance with the exemplary advantageous arrangement shown in figure 2, the two ultraviolet radiation units 8 each comprise three ultraviolet radiation sources 13 that are arranged next to each other with their respective longitudinal central axes 14 arranged parallel to a longitudinal center line 15 of the first elongated axle 9 and a longitudinal center line 16 of the second elongated axle 11. The arrangement of the ultraviolet radiation units 8 shown in figure 2 allows the fluidized solid particles 2 in the fluidization zone 7 to be effectively irradiated with UV radiation.

In accordance with another exemplary advantageous arrangement, not shown, an ultraviolet radiation unit can comprise adjacently arranged ultraviolet radiation sources each having a longitudinal center line that is arranged in a first direction that is at a non-zero angle with both the longitudinal center line of the first elongated axle and the longitudinal center line of the second elongated axle.

Based on the two exemplary advantageous arrangements mentioned above, the person skilled in the art will appreciate that the ultraviolet radiation sources can be arranged with respect to the first elongated axle and the second elongated axle in any suitable way as long as the fluidized solid particles in the fluidization zone can be effectively irradiated with UV radiation.

Furthermore, the person skilled in the art will appreciate that the number of ultraviolet radiation sources 13 shown in respective figures is exemplary in nature only. Any suitable number of ultraviolet radiation sources 13 can be applied in order to meet specific requirements of the system 1.

Figure 3 shows a schematic top view of an exemplary, non-limiting second embodiment of the system 1 according to the invention. In accordance therewith, the ultraviolet radiation unit 8 comprises ultraviolet radiation sources 13 that each have a circular cross-section and are arranged in a matrix configuration. Although the ultraviolet radiation sources 13 can have any suitable cross-section, a circular or square cross-section can be particularly advantageous for arranging the ultraviolet radiation sources 13 in a matrix configuration.

The ultraviolet radiation sources 13 can be one of mercury-arc lamps, light emitting diodes (LEDs) and laser diodes (LDs) that are adapted for emitting UV radiation having a wavelength in a range of 200 nm - 405 nm. The aforementioned types of radiation sources are examples of advantageous types of radiation sources to provide UV-C radiation having a wavelength between 200 nm and 300 nm. The highest germicidal effect can be obtained using UV-C radiation having a wavelength between 250 nm and 270 nm, with the peak wavelength for germicidal activity being 265 nm. UV curing is typically done using UV radiation having a wavelength between 365 nm and 405 nm.

Depending on the specific requirements of the system 1, the ultraviolet radiation unit 8 can be provided with any suitable combination of the aforementioned types of radiation sources.

As most clearly shown in figures 1, 4 and 5, each one of the ultraviolet radiation sources 13 is arranged inside a housing 17. At least a part of each housing 17, for example a window, that is arranged to face the interior of the process chamber 4 and is adapted to be transparent at least for ultraviolet radiation having a wavelength in the range of 200 nm - 405 nm. In this way, the housing 17 allows irradiation of the fluidized solid particles 2 that are spaced apart and tumbled around in the fluidization zone 7 in the process chamber 4 when the system 1 is in use. The housing 17 or at least a part thereof, e.g. a window, can comprise for example acrylates that are adapted to allow UV radiation to be transmitted through it.

Furthermore, instead of each ultraviolet radiation source 13 being arranged inside a dedicated housing 17, it is also possible that multiple ultraviolet radiation sources are arranged within one common housing (not shown).

Figure 1 shows that the process chamber 4 by means of example only is provided with two air knifes 18 that are configured and arranged to remove solid particles 2 from the housings 17 of the UV radiation sources 13. The person skilled in the art will appreciate that in the event that the solid particles 2 are for example granules, there generally will be little dust in the process chamber 4 upon processing the granules. The term "dust" is to be construed as residue of the granules or even the granules themselves in the case of very small granules. In that case, the application of an air knife 18 can be sufficient to remove the dust from the housing 17, i.e. from the part from the housing that is arranged to face the interior of the process chamber 4 and is adapted to allow UV radiation to be transmitted through it. The person skilled in the art will appreciate that depending on the actual arrangement and number of the housings 17 used, one air knife 18 or multiple air knives can be used to clean the housing 17 or housings in order to enable efficient irradiation of the fluidized solid particles 2 that are spaced apart in the fluidization zone 7 when the system 1 is in use.

The housing 17 can be a rotatable housing. The person skilled in the art will appreciate that in the event that the solid particles 2 are for example powder particles, there will generally be a lot of dust in the process chamber 4 upon processing the powder particles. In that case, the application of an air knife 18 in combination with a rotatable housing 17 can achieve efficient removal of the powder particles 2 from the part of the housing that is facing the interior of the process chamber 4 and is adapted to allow the UV radiation to be transmitted through it. The housing 17 can be arranged to be rotatable around the ultraviolet radiation source 13 that is stationary arranged inside the housing 17.

Figure 1 shows that the process chamber 4 is provided with a radiation intensity detection unit 19 that is configured and arranged to perform a measurement of an intensity of the ultraviolet radiation in the fluidization zone 7 during a predefined time interval. Based on this measurement it can be determined if a dose of for example UV-C radiation in the fluidization zone 7 is above a predetermined threshold to achieve disinfection of the outer surfaces of the fluidized solid particles 2 in the fluidization zone 7 and optionally initiation of chemical processes at the outer surfaces of the fluidized solid particles 2 in the fluidization zone 7. The predetermined threshold for the dose of the UV-C radiation depends on at least one of a type of microorganisms that are present on the outer surfaces of the solid particles, a distance between the ultraviolet radiation unit 8 and the fluidized solid particles 2 in the fluidization zone 7, a volume of the process chamber 4, the volume of the solid particles 2, and the outer surface area of the solid particles 2.

The person skilled in the art will appreciate that the radiation intensity detection unit 19 allows checking of the condition of the ultraviolet radiation unit 8. In this way, it can be monitored when maintenance, e.g. replacement of the ultraviolet radiation source 13 or cleaning of the ultraviolet radiation unit 8, is due. Especially in the case that the system according to the invention is configured for continuous processing, the radiation intensity detection unit 19 can be used to check if the ultraviolet radiation unit 8 remains within the required specifications during operation of the system 1. In the case that the system 1 is configured for batch processing, the radiation intensity detection unit 19 can be used to check before the start of each batch if the ultraviolet radiation unit 8 is within the required specifications. If this is the case, the batch can be started. If this is not the case, appropriate maintenance needs to be done before starting the batch.

The person skilled in the art will appreciate that any suitable kind of radiation intensity detection unit 19 can be used, for example a UV sensor. Moreover, the person skilled in the art will appreciate that any suitable number of radiation intensity detection units 19 can be applied in order to meet the specific requirements of the system 1 according to the invention.

Figure 1 shows that the process chamber 4 is provided with a surface treatment application unit 20 that is configured and arranged to apply a surface treatment composition to the outer surfaces of the fluidized solid particles 2 that are in the fluidization zone 7.

It will be clear to the person skilled in the art that a suitable surface treatment composition can be applied to the outer surfaces of the solid particles that are in the fluidization zone before or after irradiation with UV radiation. The person skilled in the art will appreciate that if a suitable surface treatment composition is applied to the outer surfaces of the solid particles after they have been disinfected or sterilized by UV-C irradiation, it is possible to prevent the outer surfaces from subsequently becoming contaminated. For example, reduced food parts that have been processed using the system according to the invention but whose outer surfaces have not been treated using a suitable surface treatment composition, can be susceptible to a subsequent contamination with salmonella. By applying a suitable surface treatment composition on the outer surfaces of the reduced food parts after irradiation thereof with UV-C radiation, the risk of such subsequent contamination of the outer surfaces can at least be reduced.

In addition, the person skilled in the art will appreciate that it is also possible that after application of a suitable surface treatment composition to the outer surfaces of the solid particles that are in the fluidization zone, said surface treatment composition can be cured using UV radiation having a wavelength between 365 nm and 405 nm.

Furthermore, the person skilled in the art will appreciate that by treating the outer surfaces with a suitable surface treatment composition it is also possible to for example reinstate microorganisms at the outer surfaces of the processed solid particles which not necessarily would have had to be removed and which may even have positive effects.

The surface treatment application unit 20 can be a spray unit that is adapted to spray the surface treatment composition on the outer surfaces of the fluidized solid particles 2 that are in the fluidization zone 7 when the system 1 is in use.

The person skilled in the art will appreciate that by using a spray unit the surface treatment composition can advantageously be applied to the outer surfaces of the fluidized solid particles 2 that are in the fluidization zone 7. The person skilled in the art will appreciate that any suitable application unit 20 can be used as long as the surface treatment composition can effectively be applied to the outer surfaces of the fluidized solid particles.

The surface treatment composition can comprise a surface sealing composition that can be cured using UV radiation having a wavelength between 365 nm and 405 nm. The person skilled in the art will appreciate that by sealing the outer surfaces of the fluidized solid particles 2 that are in the fluidization zone after for example disinfection of the outer surfaces of the fluidized solid particles 2, the risk of subsequent contamination thereof can even further be reduced.

Figure 1 also shows that the process chamber 4 of the system 1 is provided with a folding bottom 21 that is adapted to enable emptying of the process chamber 4. In this way, a residual-poor emptying of the process chamber 4 can be achieved.

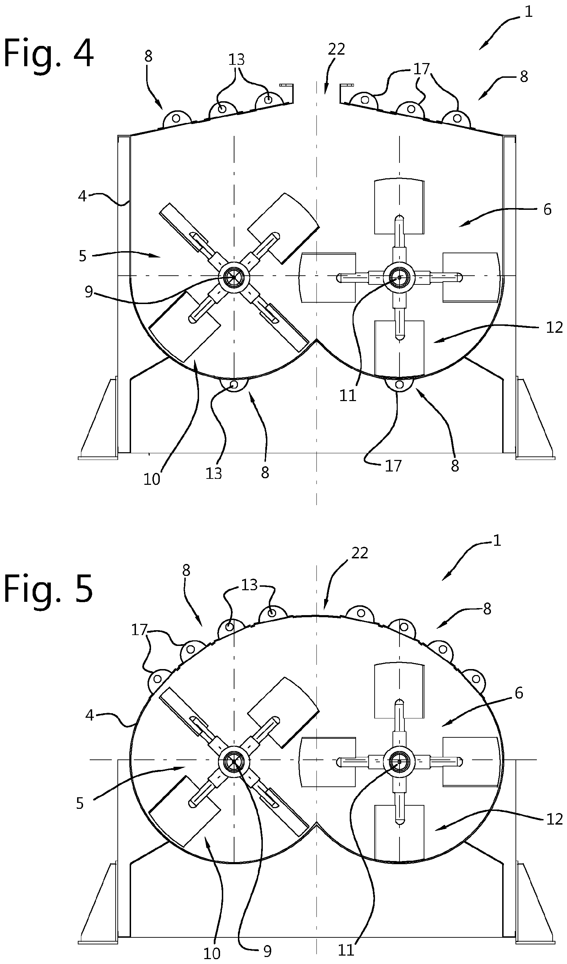

Figure 4 shows a schematic cross-sectional view of an exemplary, non-limiting third embodiment of the system 1 according to the invention in which ultraviolet radiation sources 13 are also arranged in parts of the process chamber 4 below the first activation arrangement 5 and the second activation arrangement 6. In this way, the irradiation efficiency can further be improved.

Figure 5 shows a schematic cross-sectional view of an exemplary, non-limiting fourth embodiment of the system 1 according to the invention in which the upper part of the process chamber 4 has been configured to reduce the distance between the ultraviolet radiation sources 13 and the first activation arrangement 5 and the second activation arrangement 6. Furthermore, the shape of the process chamber is adapted to improve reflection of the UV radiation. This provides another way of further improving the irradiation efficiency.

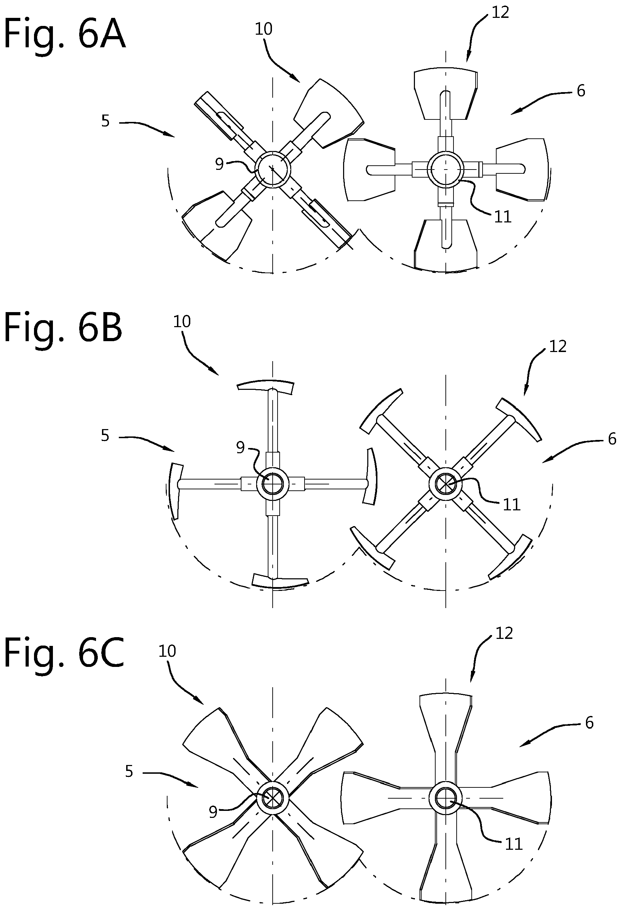

Figure 6A shows a schematic front view of an exemplary, non-limiting second embodiment of the first activation arrangement 5 and the second activation arrangement 6. In accordance with said second embodiment, the first activation arrangement 5 comprises a first elongated axle 9 that is provided with a first set of paddles 10 that have a second exemplary shape and the second activation arrangement 6 comprises a second elongated axle 11 that is provided with a second set of paddles 12 that have said second exemplary shape. The person skilled in the art will appreciate that the second exemplary shape of the first set of paddles 10 and of the second set of paddles 12 enables establishing the abovementioned fluidization zone when the system according to the invention is in use.

Figure 6B shows a schematic front view of an exemplary, non-limiting third embodiment of the first activation arrangement 5 and the second activation arrangement 6. In accordance with said third embodiment, the first activation arrangement 5 comprises a first elongated axle 9 that is provided with a first set of paddles 10 that have a third exemplary shape and the second activation arrangement 6 comprises a second elongated axle 11 that is provided with a second set of paddles 12 that have said third exemplary shape. The person skilled in the art will appreciate that the third exemplary shape of the first set of paddles 10 and of the second set of paddles 12 enables establishing the abovementioned fluidization zone when the system according to the invention is in use.

Figure 6C shows a schematic front view of an exemplary, non-limiting fourth embodiment of the first activation arrangement 5 and the second activation arrangement 6. In accordance with said fourth embodiment, the first activation arrangement 5 comprises a first elongated axle 9 that is provided with a first set of paddles 10 that have a fourth exemplary shape and the second activation arrangement 6 comprises a second elongated axle 11 that is provided with a second set of paddles 12 that have said fourth exemplary shape. The person skilled in the art will appreciate that the fourth exemplary shape of the first set of paddles 10 and of the second set of paddles 12 enables establishing the abovementioned fluidization zone when the system according to the invention is in use.

The person skilled in the art will appreciate that the first activation arrangement 5 and the second activation arrangement 6 in accordance with said second, third and fourth embodiments shown in figures 6A-6C, respectively can be arranged with respect to each other and operated when the system 1 is in use in a same way as described above in relation to the exemplary, non-limiting first embodiment shown in figures 1, 4 and 5. Consequently, the first activation arrangement 5 and the second activation arrangement 6 in accordance with said second, third and fourth embodiments can provide the same advantages, i.e. establishing a fluidization zone 7, preserving the integrity of the outer surfaces of the solid particles 2 processed using the system 1, fast mixing of different kinds of solid particles 2 of the same or different types that are dry enough to obtain mixtures comprising for example powder particles and granules, powder particles of different kinds, granules of different kinds or reduced food parts of different kinds, wherein these mixtures have a high homogeneity.

Furthermore, the person skilled in the art will appreciate that any one of the respective exemplary embodiments of the first activation arrangement 5 and the second activation arrangement 6 as shown in figures 1, 4, 5, 6A, 6B and 6C can be applied in any one of the respective exemplary embodiments of the processing system 1 shown in figures 1, 4 and 5. The actual embodiment used for the first activation arrangement 5 and the second activation arrangement 6 does not necessarily have any implication for the actual embodiment used for the processing system 1, in particular the process chamber 4, and vice versa.

The present invention can be summarized as relating to an ultraviolet radiation-based surface disinfection and/or surface processing system 1 that is adapted to disinfect outer surfaces of solid particles 2 of a stock 3 of solid particles 2 with ultraviolet radiation and optionally initiate chemical processes at outer surfaces of solid particles 2 of a stock 3 of solid particles 2 with ultraviolet radiation. The system 1 comprises a process chamber 4 for containing a stock 3 of solid particles 2. Each one of the solid particles 2 has a volume that is equal to or less than 10 cm³. The process chamber 4 is provided with two activation arrangements 5, 6 adapted to establish fluidization of the solid particles 2 of the stock 3 of solid particles 2 in a fluidization zone 7 between the two activation arrangements 5, 6. The system 1 further comprises an ultraviolet radiation unit 8 for at least partially irradiating the outer surfaces of at least the fluidized solid particles 2 in the fluidization zone 7 with ultraviolet radiation having a wavelength between 200 nm and 405 nm.

It will be clear to a person skilled in the art that the scope of the present invention is not limited to the examples discussed in the foregoing but that several amendments and modifications thereof are possible without deviating from the scope of the present invention as defined by the attached claims. In particular, combinations of specific features of various aspects of the invention may be made. An aspect of the invention may be further advantageously enhanced by adding a feature that was described in relation to another aspect of the invention. While the present invention has been illustrated and described in detail in the figures and the description, such illustration and description are to be considered illustrative or exemplary only, and not restrictive.

The present invention is not limited to the disclosed embodiments. Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. The mere fact that certain measures are recited in mutually different dependent claims does not indicate that a combination of these measures cannot be used to advantage. Any reference numerals in the claims should not be construed as limiting the scope of the present invention.

### REFERENCE NUMERALS

- 1: ultraviolet radiation-based surface disinfection and/or surface processing system
- 2: solid particles
- 3: stock of solid particles
- 4: process chamber
- 5: first activation arrangement
- 6: second activation arrangement
- 7: fluidization zone
- 8: ultraviolet radiation unit
- 9: first elongated axle
- 10: first set of paddles
- 11: second elongated axle
- 12: second set of paddles
- 13: ultraviolet radiation source
- 14: longitudinal center line of the ultraviolet radiation source
- 15: longitudinal center line of the first elongated axle
- 16: longitudinal center line of the second elongated axle
- 17: housing
- 18: air knife
- 19: radiation intensity detection unit
- 20: surface treatment application unit
- 21: folding bottom
- 22: inlet opening of process chamber

## Claims

1. An ultraviolet radiation-based surface disinfection system (1) that is adapted to disinfect outer surfaces of solid particles (2) of a stock (3) of solid particles (2) with ultraviolet radiation, said system (1) comprising a process chamber (4) that contains a stock (3) of solid particles (2) dry enough to allow fluidization of the solid particles, wherein each one of the solid particles (2) of the stock (3) of solid particles (2) has a volume that is equal to or less than 10 cm³,
wherein the process chamber (4) is provided with:
• a first activation arrangement (5) and a second activation arrangement (6),
wherein the first activation arrangement (5) comprises a first elongated axle (9) that is provided with a first set of paddles (10),
wherein the second activation arrangement (6) comprises a second elongated axle (11) that is provided with a second set of paddles (12),
wherein the first elongated axle (9) and the second elongated axle (11) are parallel with respect to each other and configured and arranged to simultaneously rotate in opposite directions,
wherein the first set of paddles (10) and the second set of paddles (12) are configured to interact, when said system (1) is in use, with the solid particles (2) of the stock (3) of solid particles (2) in the process chamber (4) to establish fluidization of solid particles (2) of the stock (3) of solid particles (2) in a fluidization zone (7) between the first activation arrangement (5) and the second activation arrangement (6) such that the fluidized solid particles (2) are spaced apart and tumbled around in the fluidization zone (7); and
• an ultraviolet radiation unit (8) that is configured to emit ultraviolet radiation having a wavelength in a range of 200 nm - 300 nm, wherein the ultraviolet radiation unit (8) is arranged to at least partially irradiate the outer surfaces of at least the fluidized solid particles (2) in the fluidization zone (7).

2. The system (1) according to claim 1, wherein the solid particles are reduced food parts.

3. The system (1) according to claim 2, wherein the ultraviolet radiation unit (8) comprises an ultraviolet radiation source (13) that has a longitudinal center line (14), wherein the longitudinal center line (14) of the ultraviolet radiation source (13) is arranged parallel to a longitudinal center line (15) of the first elongated axle (9) and a longitudinal center line (16) of the second elongated axle (11).

4. The system (1) according to claim 2, wherein the ultraviolet radiation unit (8) comprises an ultraviolet radiation source (13) that has a longitudinal center line (14), wherein the longitudinal center line (14) of the ultraviolet radiation source (13) is arranged in a first direction that is at a non-zero angle with both a longitudinal center line (15) of the first elongated axle (9) and a longitudinal center line (16) of the second elongated axle (11).

5. The system (1) according to claim 1 or 2, wherein the ultraviolet radiation unit (8) comprises an ultraviolet radiation source (13) that has a circular or a square cross-section.

6. The system according to any one of the claims 3-5, wherein the ultraviolet radiation source (13) is one of a mercury-arc lamp, a light emitting diode (LED) and a laser diode (LD) being adapted to emit ultraviolet radiation having a wavelength in the range of 200 nm - 405 nm.

7. The system (1) according to any one of the claims 3-6, wherein the ultraviolet radiation source (13) is arranged inside a housing (17) that is transparent at least for ultraviolet radiation having a wavelength in the range of 200 nm - 405 nm.

8. The system (1) according to claim 7, wherein the process chamber (4) is provided with an air knife (18) that is configured and arranged to remove solid particles from the housing (17).

9. The system (1) according to claim 7 or 8, wherein the housing (17) is a rotatable housing (17).

10. The system (1) according to any one of the preceding claims, wherein the process chamber (4) is provided with a radiation intensity detection unit (19) that is configured and arranged to perform a measurement of an intensity of the ultraviolet radiation in the fluidization zone (7) during a predefined time interval and to determine based on said measurement if a dose of the ultraviolet radiation in the fluidization zone (7) is above a predetermined threshold to achieve disinfection of the outer surfaces of the fluidized solid particles (2) in the fluidization zone (7) and/or initiation of chemical processes at the outer surfaces of the fluidized solid particles (2) in the fluidization zone (7), wherein the predetermined threshold for the dose of the ultraviolet radiation is dependent on at least one of a type of microorganisms that are present on the outer surfaces of the solid particles (2), a distance between the ultraviolet radiation unit (8) and the fluidized solid particles (2) in the fluidization zone (7), a volume of the process chamber (4), the volume of the solid particles (2), and the outer surface area of the solid particles (2).

11. The system (1) according to any one of the preceding claims, wherein the process chamber (4) has an inner surface having an arithmetic average surface roughness Ra that is equal to or less than 0.8 µm.

12. The system (1) according to any one of the preceding claims, wherein the process chamber (4) is provided with a surface treatment application unit (20) that is configured and arranged to apply a surface treatment composition to the outer surfaces of the fluidized solid particles (2) that are in the fluidization zone (7).

13. The system (1) according to claim 12, wherein the surface treatment application unit (20) is a spray unit that is adapted to spray the surface treatment composition on the outer surfaces of the fluidized solid particles (2) that are in the fluidization zone (7).

14. The system (1) according to any one of the preceding claims, wherein the process chamber (4) is provided with a folding bottom (21) that is adapted to enable emptying of the process chamber (4).

15. The system (1) according to any one of the preceding claims, wherein the process chamber (4) is configured to receive different kinds of solid particles that all have outer surfaces that are to be disinfected using ultraviolet radiation having a wavelength in a range of 200 nm - 300 nm while the solid particles are being mixed in the fluidization zone (7) and/or to be irradiated using ultraviolet radiation having a wavelength in a range of 365 nm - 405 nm to initiate chemical processes thereon while the solid particles are being mixed in the fluidization zone (7).

## Patentansprüche

1. Auf ultravioletter Strahlung basierendes Oberflächendesinfektionssystem (1), das angepasst ist, um Außenoberflächen von Feststoffteilchen (2) eines Bestandes (3) aus Feststoffteilchen (2) mit Ultraviolettstrahlung zu desinfizieren, das System (1) umfassend eine Prozesskammer (4), die ein Bestandes (3) aus Feststoffteilchen (2) enthält, das ausreichend trocken ist, um eine Fluidisierung der Feststoffteilchen zuzulassen, wobei jedes eine der Feststoffteilchen (2) des Bestandes (3) aus Feststoffteilchen (2) ein Volumen, das gleich oder kleiner als 10 cm³ ist, aufweist,
wobei die Prozesskammer (4) versehen ist mit:
• einer ersten Aktivierungsanordnung (5) und einer zweiten Aktivierungsanordnung (6),
wobei die erste Aktivierungsanordnung (5) eine erste längliche Achse (9), die mit einem ersten Satz Paddel (10) versehen ist, umfasst,
wobei die zweite Aktivierungsanordnung (6) eine zweite längliche Achse (11), die mit einem zweiten Satz Paddel (12) versehen ist, umfasst,
wobei die erste längliche Achse (9) und die zweite längliche Achse (11) parallel bezüglich zueinander und konfiguriert und angeordnet sind, um sich in entgegengesetzte Richtungen gleichzeitig zu drehen,
wobei der erste Satz Paddel (10) und der zweite Satz Paddel (12) konfiguriert sind, um, wenn das System (1) in Verwendung ist, mit den Feststoffteilchen (2) des Bestandes (3) aus Feststoffteilchen (2) in der Prozesskammer (4) zu interagieren, um die Fluidisierung von Feststoffteilchen (2) des Bestandes (3) aus Feststoffteilchen (2) in einer Fluidisierungszone (7) zwischen der ersten Aktivierungsanordnung (5) und der zweiten Aktivierungsanordnung (6) derart zu erzeugen, dass die fluidisierten Feststoffteilchen (2) in der Fluidisierungszone (7) voneinander beabstandet und durcheinander geworfen sind; und
• eine Ultraviolettstrahlungseinheit (8), die konfiguriert ist, um Ultraviolettstrahlung, die eine Wellenlänge in einem Bereich von 200 nm - 300 nm aufweist, zu emittieren, wobei die Ultraviolettstrahlungseinheit (8) angeordnet ist, um die Außenoberflächen von mindestens den fluidisierten Feststoffteilchen (2) in der Fluidisierungszone (7) mindestens teilweise zu bestrahlen.

2. System (1) nach Anspruch 1, wobei die Feststoffteilchen reduzierte Lebensmittelteile sind.

3. System (1) nach Anspruch 2, wobei die Ultraviolettstrahlungseinheit (8) eine Ultraviolettstrahlungsquelle (13), die eine Längsmittellinie (14) aufweist, umfasst, wobei die Längsmittellinie (14) der Ultraviolettstrahlungsquelle (13) parallel zu einer Längsmittellinie (15) der ersten länglichen Achse (9) und einer Längsmittellinie (16) der zweiten länglichen Achse (11) angeordnet ist.

4. System (1) nach Anspruch 2, wobei die Ultraviolettstrahlungseinheit (8) eine Ultraviolettstrahlungsquelle (13), die eine Längsmittellinie (14) aufweist, umfasst, wobei die Längsmittellinie (14) der Ultraviolettstrahlungsquelle (13) in einer ersten Richtung, die in einem Winkel ungleich null mit sowohl einer Längsmittellinie (15) der ersten länglichen Achse (9) als auch einer Längsmittellinie (16) der zweiten länglichen Achse (11) ist, angeordnet ist.

5. System (1) nach Anspruch 1 oder 2, wobei die Ultraviolettstrahlungseinheit (8) eine Ultraviolettstrahlungsquelle (13), die einen kreisförmigen oder einen quadratischen Querschnitt aufweist, umfasst.

6. System nach einem der Ansprüche 3 bis 5, wobei die Ultraviolettstrahlungsquelle (13) eine von einer Quecksilberdampflampe, einer lichtemittierenden Diode (LED) und einer Laserdiode (LD) ist, die angepasst ist, um Ultraviolettstrahlung, die eine Wellenlänge in dem Bereich von 200 nm - 405 nm aufweist, zu emittieren.

7. System (1) nach einem der Ansprüche 3 bis 6, wobei die Ultraviolettstrahlungsquelle (13) innerhalb eines Gehäuses (17) angeordnet ist, das mindestens für Ultraviolettstrahlung, die eine Wellenlänge in dem Bereich von 200 nm - 405 nm aufweist, transparent ist.

8. System (1) nach Anspruch 7, wobei die Prozesskammer (4) mit einem Luftmesser (18) versehen ist, das konfiguriert und angeordnet ist, um Feststoffteilchen von dem Gehäuse (17) zu entfernen.

9. System (1) nach Anspruch 7 oder 8, wobei das Gehäuse (17) ein drehbares Gehäuse (17) ist.

10. System (1) nach einem der vorstehenden Ansprüche, wobei die Prozesskammer (4) mit einer Strahlungsintensitätserfassungseinheit (19) versehen ist, die konfiguriert und angeordnet ist, um eine Messung einer Intensität der Ultraviolettstrahlung in der Fluidisierungszone (7) während eines vordefinierten Zeitintervalls durchzuführen und basierend auf der Messung zu bestimmen, ob eine Dosis der Ultraviolettstrahlung in der Fluidisierungszone (7) über einem zuvor bestimmten Schwellenwert ist, um eine Desinfektion der Außenflächen der fluidisierten Feststoffpartikel (2) zu erreichen) in der Fluidisierungszone (7) und/oder Auslösung chemischer Prozesse an den Außenflächen der fluidisierten Feststoffpartikel (2) in der Fluidisierungszone (7), wobei der vorgegebene Schwellenwert für die Dosis der ultravioletten Strahlung von at abhängt mindestens einer einer Art von Mikroorganismen, die auf den Außenflächen des vorhanden sind Feststoffpartikel (2), ein Abstand zwischen der UV-Strahlungseinheit (8) und den fluidisierten Feststoffpartikeln (2) in der Fluidisierungszone (7), ein Volumen der Prozesskammer (4), das Volumen der Feststoffpartikel (2) und der äußeren Oberfläche der Feststoffpartikel (2).

11. System (1) nach einem der vorstehenden Ansprüche, wobei die Prozesskammer (4) eine Innenoberfläche aufweist, die eine arithmetische durchschnittliche Oberflächenrauheit Ra, die gleich oder kleiner als 0,8 µm ist, aufweist.

12. System (1) nach einem der vorstehenden Ansprüche, wobei die Prozesskammer (4) mit einer Oberflächenbehandlungsautragseinheit (20) versehen ist, die konfiguriert und angeordnet ist, um eine Oberflächenbehandlungszusammensetzung auf die Außenoberflächen der fluidisierten Feststoffteilchen (2), die in der Fluidisierungszone (7) sind, aufzutragen.

13. System (1) nach Anspruch 12, wobei die Oberflächenbehandlungsautragseinheit (20) eine Sprüheinheit ist, die angepasst ist, um die Oberflächenbehandlungszusammensetzung auf die Außenoberflächen der fluidisierten Feststoffteilchen (2), die in der Fluidisierungszone (7) sind, zu sprühen.

14. System (1) nach einem der vorstehenden Ansprüche, wobei die Prozesskammer (4) mit einem Faltboden (21) versehen ist, der angepasst ist, um ein Entleeren der Prozesskammer (4) zu ermöglichen.

15. System (1) nach einem der vorstehenden Ansprüche, wobei die Prozesskammer (4) konfiguriert ist, dass sie verschiedene Arten von Feststoffpartikeln aufnimmt, die alle äußere Oberflächen haben, die mit ultravioletter Strahlung mit einer Wellenlänge in einem Bereich von zu desinfizieren sind 200 nm-300 nm, während die Feststoffpartikel in der Fluidisierungszone (7) gemischt werden, und/oder mit ultravioletter Strahlung mit einer Wellenlänge im Bereich von 365 nm-405 nm bestrahlt zu werden, um darauf chemische Prozesse auszulösen, während die Feststoffpartikel in der Fluidisierungszone (7) in der Fluidisierungszone (7) gemischt werden.

## Revendications

1. Système de désinfection de surface à base de rayonnement ultraviolet (1) qui est adapté pour désinfecter des surfaces externes de particules solides (2) d'un stock (3) de particules solides (2) avec un rayonnement ultraviolet, ledit système (1) comprenant une chambre de traitement (4) qui contient un stock (3) de particules solides (2) suffisamment sèches pour permettre une fluidisation des particules solides, dans lequel chacune des particules solides (2) du stock (3) de particules solides (2) a un volume qui est égal ou inférieur à 10 cm³, dans lequel la chambre de traitement (4) est pourvue de :
• un premier agencement d'activation (5) et un second agencement d'activation (6),
dans lequel le premier agencement d'activation (5) comprend un premier essieu allongé (9) qui est pourvu d'un premier ensemble de pales (10),
dans lequel le second agencement d'activation (6) comprend un second essieu allongé (11) qui est pourvu d'un second ensemble de pales (12),
dans lequel le premier essieu allongé (9) et le second essieu allongé (11) sont parallèles l'un par rapport à l'autre et configurés et agencés pour tourner simultanément dans des sens opposés,
dans lequel le premier ensemble de pales (10) et le second ensemble de palettes (12) sont configurés pour interagir, lorsque ledit système (1) est en cours d'utilisation, avec les particules solides (2) du stock (3) de particules solides (2) dans la chambre de traitement (4) pour établir une fluidisation de particules solides (2) du stock (3) de particules solides (2) dans une zone de fluidisation (7) entre le premier agencement d'activation (5) et le second agencement d'activation (6) de telle sorte que les particules solides (2) fluidisées sont espacées et remuées dans la zone de fluidisation (7) ; et
• une unité de rayonnement ultraviolet (8) qui est configurée pour émettre un rayonnement ultraviolet ayant une longueur d'onde comprise dans une plage de 200 nm à 300 nm, dans lequel l'unité de rayonnement ultraviolet (8) est agencée pour irradier au moins partiellement les surfaces externes d'au moins les particules solides (2) fluidisées dans la zone de fluidisation (7).

2. Système (1) selon la revendication 1, dans lequel les particules solides sont des morceaux d'aliments de taille réduite.

3. Système (1) selon la revendication 2, dans lequel l'unité de rayonnement ultraviolet (8) comprend une source de rayonnement ultraviolet (13) qui a une ligne centrale longitudinale (14), dans lequel la ligne centrale longitudinale (14) de la source de rayonnement ultraviolet (13) est agencée parallèlement à une ligne centrale longitudinale (15) du premier essieu allongé (9) et à une ligne centrale longitudinale (16) du second essieu allongé (11).

4. Système (1) selon la revendication 2, dans lequel l'unité de rayonnement ultraviolet (8) comprend une source de rayonnement ultraviolet (13) qui a une ligne centrale longitudinale (14), dans lequel la ligne centrale longitudinale (14) de la source de rayonnement ultraviolet (13) est agencée dans une première direction qui est à un angle non nul avec à la fois une ligne centrale longitudinale (15) du premier essieu allongé (9) et une ligne centrale longitudinale (16) du second essieu allongé (11).

5. Système (1) selon la revendication 1 ou 2, dans lequel l'unité de rayonnement ultraviolet (8) comprend une source de rayonnement ultraviolet (13) qui a une section transversale circulaire ou carrée.

6. Système selon l'une quelconque des revendications 3 à 5, dans lequel la source de rayonnement ultraviolet (13) est l'une parmi une lampe à arc au mercure, une diode électroluminescente (DEL) et une diode laser (LD) adaptée pour émettre un rayonnement ultraviolet ayant une longueur d'onde comprise dans la plage de 200 nm à 405 nm.

7. Système (1) selon l'une quelconque des revendications 3 à 6, dans lequel la source de rayonnement ultraviolet (13) est agencée à l'intérieur d'un boîtier (17) qui est transparent au moins pour un rayonnement ultraviolet ayant une longueur d'onde comprise dans la plage de 200 nm à 405 nm.

8. Système (1) selon la revendication 7, dans lequel la chambre de traitement (4) est pourvue d'une lame d'air (18) qui est configurée et agencée pour retirer des particules solides du boîtier (17).

9. Système (1) selon la revendication 7 ou 8, dans lequel le boîtier (17) est un boîtier (17) rotatif.

10. Système (1) selon l'une quelconque des revendications précédentes, dans lequel la chambre de traitement (4) est pourvue d'une unité de détection d'intensité de rayonnement (19) qui est configurée et agencée pour effectuer une mesure d'une intensité du rayonnement ultraviolet dans la zone de fluidisation (7) pendant un intervalle de temps prédéfini et pour déterminer sur la base de ladite mesure si une dose du rayonnement ultraviolet dans la zone de fluidisation (7) est supérieure à un seuil prédéterminé pour obtenir une désinfection des surfaces externes des particules solides (2) fluidisées dans la zone de fluidisation (7) et/ou une initiation de processus chimiques au niveau des surfaces externes des particules solides (2) fluidisées dans la zone de fluidisation (7), dans lequel le seuil prédéterminé pour la dose du rayonnement ultraviolet dépend d'au moins l'un parmi un type de micro-organismes qui sont présents sur les surfaces externes des particules solides (2), une distance entre l'unité de rayonnement ultraviolet (8) et les particules solides (2) fluidisées dans la zone de fluidisation (7), un volume de la chambre de traitement (4), le volume des particules solides (2), et la surface active externe des particules solides (2).

11. Système (1) selon l'une quelconque des revendications précédentes, dans lequel la chambre de traitement (4) a une surface interne ayant une rugosité de surface moyenne arithmétique Ra qui est égale ou inférieure à 0,8 µm.

12. Système (1) selon l'une quelconque des revendications précédentes, dans lequel la chambre de traitement (4) est pourvue d'une unité d'application de traitement de surface (20) qui est configurée et agencée pour appliquer une composition de traitement de surface aux surfaces externes des particules solides (2) fluidisées qui se trouvent dans la zone de fluidisation (7).

13. Système (1) selon la revendication 12, dans lequel l'unité d'application de traitement de surface (20) est une unité de pulvérisation qui est adaptée pour pulvériser la composition de traitement de surface sur les surfaces externes des particules solides (2) fluidisées qui se trouvent dans la zone de fluidisation (7).

14. Système (1) selon l'une quelconque des revendications précédentes, dans lequel la chambre de traitement (4) est pourvue d'une partie inférieure pliante (21) qui est adaptée pour permettre de vider la chambre de traitement (4).

15. Système (1) selon l'une quelconque des revendications précédentes, dans lequel la chambre de traitement (4) est configurée pour recevoir différentes sortes de particules solides qui ont toutes des surfaces externes qui doivent être désinfectées à l'aide d'un rayonnement ultraviolet ayant une longueur d'onde comprise dans une plage de 200 nm à 300 nm tandis que les particules solides sont en cours de mélange dans la zone de fluidisation (7) et/ou irradiées à l'aide d'un rayonnement ultraviolet ayant une longueur d'onde comprise dans une plage de 365 nm à 405 nm pour initier des processus chimiques sur celles-ci alors que les particules solides sont en cours de mélange dans la zone de fluidisation (7).
